# EUROPÄISCHE PATENTSCHRIFT

(11) **EP 1 484 039 B1**
(45) Veröffentlichungstag und Bekanntmachung des Hinweises auf die Patenterteilung: **20.06.2007**
(21) Anmeldenummer: 04012160.0
(22) Anmeldetag: 22.05.2004
(51) Int. Cl.: A61F 5/00, A61B 17/135

(54) **Einrichtung zur Erzeugung einer künstlichen Verengung im Gastro-Intestinal-Trakt**
An apparatus for creating an artificial reduction in the gastrointestinal tract
Dispositif pour générer une constriction artificielle du tract gastro-intestinal

(30) Priorität: 04.06.2003 AT 8622003
(43) Veröffentlichungstag der Anmeldung: 08.12.2004
(73) Patentinhaber: AMI Agency for Medical Innovations GmbH, 6840 Götzis (AT)
(72) Erfinder: Egle, Walter, 6842 Koblach (AT)
(74) Vertreter: Hofmann, Ralf U.

(56) Entgegenhaltungen:
- EP-A- 1 389 453
- WO-A-20/04019671
- US-A1- 2002 198 548

## Beschreibung

Die Erfindung betrifft eine Einrichtung zur Erzeugung einer künstlichen Verengung im Gastro-Intestinal-Trakt mit einem ringförmig um den jeweiligen Teil des Gastro-Intestinal-Traktes legbaren Band und einer Verschlusseinrichtung zum Verbinden der Endbereiche des ringförmig um den Teil des Gastro-Intestinal-Traktes gelegten Bandes, welches im geschlossenen Zustand der Verschlusseinrichtung eine Durchtrittsöffnung umschließt, wobei das Band eine mit einem Füllmedium befüllbare Hohlkammer aufweist, die sich über zumindest einen Großteil der Länge des Bandes erstreckt, und bei einer Befüllung der Hohlkammer eine die Durchtrittsöffnung begrenzende Wand des Bandes sich unter Verkleinerung der Durchtrittsöffnung in Richtung zur Achse der Durchtrittsöffnung ausdehnt, und wobei die Einrichtung weiters einen mit dem Band verbundenen Schaumstoffkörper umfasst.

Eine Einrichtung zur Erzeugung einer künstlichen Verengung im Gastro-Intestinal-Trakt ist beispielsweise in Form eines Magenbandes aus der EP 0 702 529 B1 bekannt. Diese Einrichtung weist ein um den Mageneingang legbares Band auf, welches mit einer längs verlaufenden inneren Hohlkammer ausgebildet ist. Zum Verschließen des um den Mageneingang ringförmig gelegten Bandes weist dieses eine Verschlusseinrichtung mit einem am einen Ende des Bandes angeordneten und eine Einstecköffnung aufweisenden ersten Verschlussteil und einem am anderen Ende des Bandes angeordneten, durch die Einstecköffnung einführbaren und gegenüber dieser verrastbaren zweiten Verschlussteilauf. Zur Verengung des Durchlassquerschnittes der Durchtrittsöffnung des Bandes und somit des Mageneinganges wird die Hohlkammer des Bandes mit einem Füllmedium befüllt, wobei die Menge des Füllmediums vom gewünschten Durchlassquerschnitt abhängt. Zur Befüllung des Bandes mit dem Füllmedium ist ein mit dem Band verbundener Injektionsport vorgesehen, der unter die Haut des Patienten implantiert wird.

Neben einer Ausbildung als Magenband kann eine Einrichtung dieser Art insbesondere auch als Analband zum Verschluss eines, gegebenenfalls künstlichen, Anus ausgebildet sein.

Eine Einrichtung der eingangs genannten Art ist aus der US 6,511,490 B2 bekannt. Die Einrichtung weist einen die Durchtrittsöffnung umgebenden Schaumstoffkörper mit einer rauen Oberfläche auf, beispielsweise aus einem offenzelligen Polyurethan- oder Silikonschaum. Es soll dadurch ein Verrutschen der um den Magen angelegten Einrichtung verhindert werden. Der Schaumstoffkörper ist an der der Durchtrittsöffnung zugewandten Seitenfläche des befüllbaren Bandes angebracht.

Aus der US 4,592,339 ist eine Magenband bekannt, welches einen mehrlagigen Bandabschnitt aufweist. Über einen Teil der Längserstreckung des Bandabschnittes ist zwischen dessen Lagen ein aufblasbarer Ballon angeordnet, mit welchem die Durchtrittsöffnung des Magenbandes verkleinert werden kann.

Ein weiteres Magenband ist aus der WO 03/020183 A1 bekannt. Die Durchtrittsöffnung dieses Magenbandes kann insbesondere mittels einer motorischen Stelleinheit verändert werden, die mit einem Endabschnitt des Bandes zusammenwirkt. Das Band wird von einer Lage aus einem viskoelastischen Material umgeben, um die Magenwand zu schonen. Das viskoelastische Material kann einen Schaumstoff umfassen.

Ein Problem bei Einrichtungen zur Erzeugung einer künstlichen Verengung im Gastro-Intestinal-Trakt, welche ein Band mit einer befüllbaren Hohlkammer aufweist, besteht darin, dass diese früher oder später im Laufe ihres Gebrauchs leck werden können, so dass ihre Funktion nicht mehr gegeben ist. Es ist in der Folge eine operative Entfernung und Ersetzung des Bandes erforderlich, was mit einer entsprechenden Belastung des Patienten verbunden ist. Solche Lecks treten in der Praxis insbesondere in der das Band zur Durchtrittsöffnung hin begrenzenden Membran auf. Solche Lecks können beispielsweise durch Materialermüdung im Laufe des Gebrauchs oder durch eine Überfüllung des Bandes auftreten. Für sogenannte "frühe" Lecks, die bis etwa ein Jahr nach dem Einsetzen des Bandes auftreten, sind meist Verletzungen verantwortlich, die bei der operativen, insbesondere laparoskopischen, Platzierung des Bandes durch ein chirurgisches Instrument erfolgt sind.

Aufgabe der Erfindung ist es, eine verbesserte Einrichtung der eingangs genannten Art bereitzustellen, bei welcher die Häufigkeit von operativen Eingriffen, welche aufgrund eines Lecks des Bandes notwendig werden, verringert wird. Erfindungsgemäß gelingt dies durch eine Einrichtung mit den Merkmalen des Anspruchs 1.

Durch die erfindungsgemäße Ausbildung kann eine wesentlich verbesserte Lecksicherheit erreicht werden, wobei die Dichtheit der Hohlkammer normalerweise auch noch bei einer Verletzung der die Durchtrittsöffnung begrenzenden Wand gegeben ist.

In einer vorteilhaften Ausführungsform der Erfindung besteht der Schaumstoffkörper aus Silikonschaum.

Als Füllmedium kann beispielsweise steriles Wasser eingesetzt werden. Zur weiteren Verbesserung der Lecksicherheit kann auch eine Flüssigkeit mit einer höheren Viskosität eingesetzt werden, beispielsweise Silikongel.

Weitere Vorteile und Einzelheiten der Erfindung werden im Folgenden anhand der beiliegenden Zeichnung erläutert. In dieser zeigen:
- Fig. 1: eine Ansicht eines Ausführungsbeispiels der Erfindung;
- Fig. 2: eine Seitenansicht, teilweise im Schnitt entlang der Linie AA von Fig. 1;
- Fig. 3: einen vergrößerten Ausschnitt aus Fig. 2;
- Fig. 4: einen Schnitt entlang der Linie BB von Fig. 3;
- Fig. 5: eine perspektivische Darstellung des Bandes mit der Verschlusseinrichtung und
- Fig. 6: eine schematische Darstellung einer modifizierten Ausführungsform in einem Fig. 4 entsprechenden Schnitt.

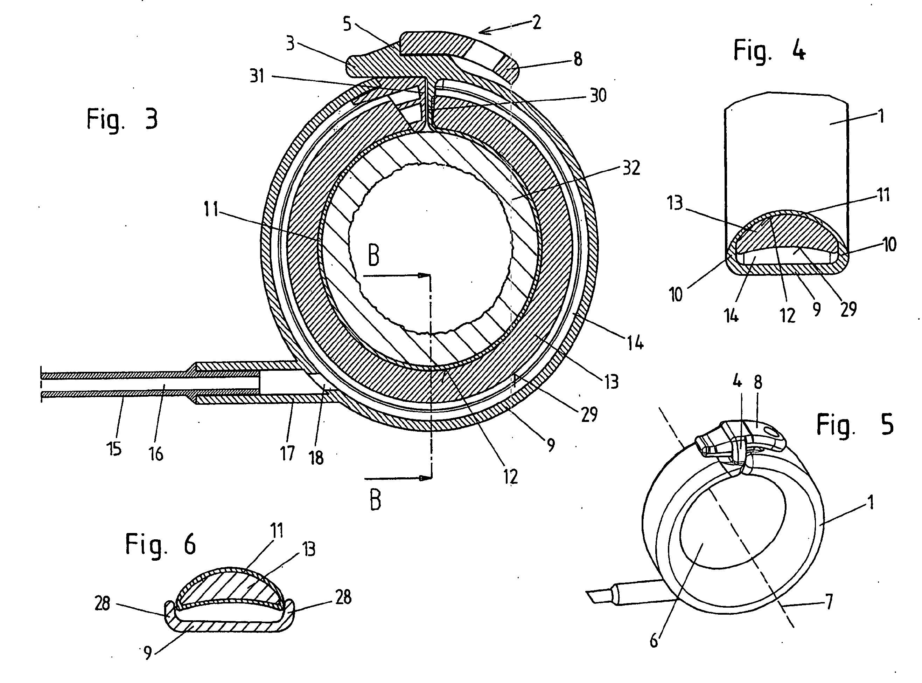

Das in den Figuren dargestellte Ausführungsbeispiel einer erfindungsgemäßen Einrichtung ist als Magenband ausgebildet und weist ein ringförmig um den entsprechenden, in Fig. 3 nur schematisch im Querschnitt angedeuteten, Teil 32 des Gastro-Intestinal-Traktes, hier den Magen, den Mageneingang oder die Speiseröhre zu legendes Band 1 auf. Das Band ist im Bereich seiner beiden Enden mit Teilen einer Verschlusseinrichtung 2 versehen. Die Verschlusseinrichtung weist einen am einen Ende des Bandes 1 angebrachten Fortsatz 3 und einen am anderen Ende des Bandes angebrachten Steg 4 mit einer Einstecköffnung zum Durchstecken des Fortsatzes 3 auf. Zum Schließen der Verschlusseinrichtung 2 wird der Fortsatz 3 durch die Einstecköffnung im Steg 4 durchgezogen, wobei ein Rastvorsprung 5 am Fortsatz 3 mit dem Steg 4 verrastet und die Verschlusseinrichtung gegen ein ungewolltes Öffnen sichert.

Im geschlossenen Zustand der Verschlusseinrichtung 2 umschließt das dann ringförmige Band 1 eine Durchtrittsöffnung 6.

Der Rastvorsprung 5 springt vorzugsweise über die der Durchtrittsöffnung 6 abgewandte Seite und die in Richtung der Achse 7 der Durchtrittsöffnung 6 weisenden Randseiten des Fortsatzes 3 vor, wobei der Rastvorsprung 5 eine vom freien Ende des Fortsatzes 3 aus gesehen konisch sich verbreiternde Form aufweist, um ein Durchziehen durch die Einstecköffnung des Steges 4 zu ermöglichen. Auf der Seite, welche im eingesteckten Zustand den Rand der Einstecköffnung des Steges 4 hintergreift, ist der Rastvorsprung 5 stufenförmig ausgebildet und steht etwa senkrecht vom Fortsatz 3 ab.

Vorzugsweise sind der Fortsatz 3 mit seinem Rastvorsprung 5 und der Steg 4 aus einem elastischen Material, insbesondere Silikon, mit einer solchen Härte und weiters mit einer solchen zusammenwirkenden Geometrie ausgebildet, dass die Verschlusseinrichtung bei einem Aufreißzug, der einen vorgegebenen Grenzwert überschreitet, sich öffnet. Es kann dadurch ein Sicherheitsverschluss bereitgestellt werden, durch welchen ein unzulässiger hoher Druck im umschlossenen Körperorgan vermieden werden kann.

Vom Steg 4 steht eine Zuglasche 8 in eine der Einsteckrichtung des Fortsatzes 3 etwa entgegenstehende Richtung ab, wodurch durch Angriff von medizinischen Instrumenten einerseits an der Zuglasche, andererseits am mit seinem vorderen Ende durch die Einstecköffnung im Steg 4 geführten Fortsatz 3 das Schließen der Verschlusseinrichtung 2 erleichtert wird.

Die an den beiden Enden des Bandes 1 angeordneten Teile der Verschlusseinrichtung 2 können an Pfropfen angebracht sein, welche in die Enden des Bandes ragen, in diese Enden eingeklebt sind und diese Enden verschließen. Auch eine materialeinstückige Ausbildung der Verschlusseinrichtung oder Teilen hiervon mit dem Band 1, insbesondere auf der Seite des Fortsatzes 3 ist denkbar und möglich.

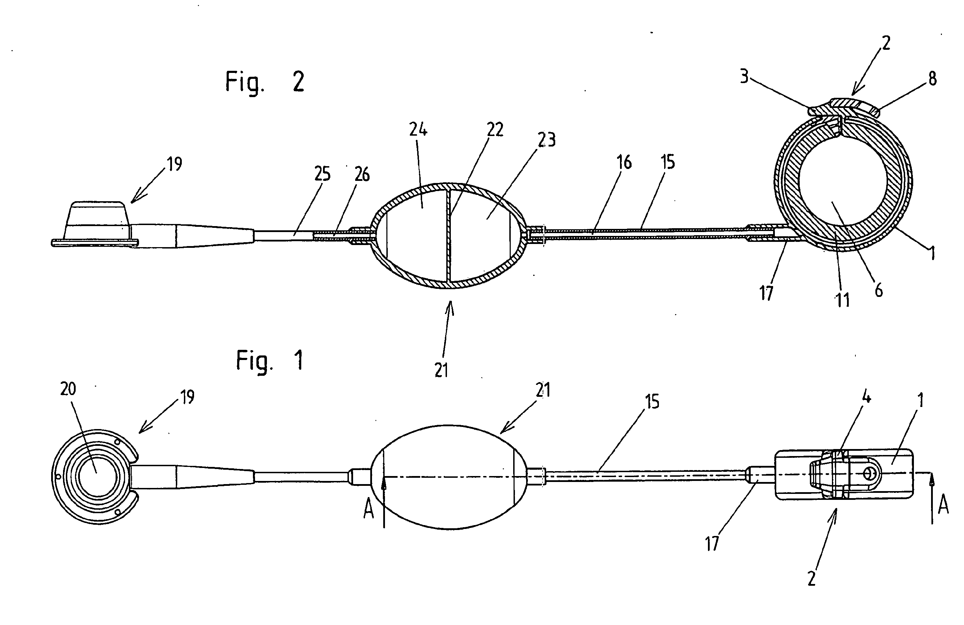

Das Band weist beim in den Fig. 1 bis 5 dargestellten Ausführungsbeispiel einen umfangsgeschlossenen Schlauch mit einem von der Durchtrittsöffnung 6 abgewandten Rückenabschnitt 9 und einer auf der Seite der Durchtrittsöffnung 6 gelegenen und die Durchtrittsöffnung 6 begrenzenden Wand (bzw. Haut oder Membran) 11 auf. Der Rückenabschnitt 9 weist eine größere Dicke als die Wand 11 auf, wodurch seine elastische Dehnbarkeit geringer ist. Weiters kann der Rückenabschnitt 9 auch mit einer (in den Fig. nicht dargestellten) Verstärkungslage versehen sein, die beispielsweise in das Material des Rückenabschnitts 9 eingebettet sein kann oder auf die von der Durchtrittsöffnung 6 abgewandte Oberfläche des Rückenabschnitts 9 aufgeklebt sein kann. Eine solche Verstärkungslage kann in Längsrichtung und vorteilhafterweise auch in Querrichtung über ihre gesamte Ausdehnung durchgehende Fäden aufweisen und insbesondere als Rechteckgewebe, beispielsweise aus einem Kunststoffmaterial ausgebildet sein.

Zwischen dem Rückenabschnitt 9 und der Wand 11 sind von den beiden Randseiten des Rückenabschnitts 9 in Richtung zur Durchtrittsöffnung 6 ausgehende Verbindungsabschnitte 10 angeordnet, deren Materialdicke ebenfalls größer ist als die der Wand 11. Prinzipiell denkbar und möglich wäre es auch, dass diese Verbindungsabschnitte 10 entfallen.

An die von der Durchtrittsöffnung 6 abgewandte Oberfläche 12 der Wand 11 grenzt ein Schaumstoffkörper 13 an, welcher bevorzugterweise aus Silikonschaum besteht. Dieser Schaumstoffkörper 13, der sich über die gesamte Länge der die Durchtrittsöffnung 6 begrenzenden Wand 11 erstreckt, ist mit der Wand 11 zumindest über einen Großteil seiner an der Wand anliegenden Fläche mit dieser verklebt. Vorzugsweise ist der Schaumstoffkörper 13 über die gesamte Länge der Wand 11 vollflächig mit dieser verklebt. Zwischen der von der Durchtrittsöffnung 6 abgewandten Oberfläche 29 des Schaumstoffkörpers 13 und dem Rückenabschnitt 9 erstreckt sich eine Hohlkammer 14 über zumindest einen Großteil der Länge des Bandes 1, vorzugsweise über die gesamte Länge des Bandes 1. Die Hohlkammer 14 ist somit auf ihrer der Durchtrittsöffnung 6 zugewandten Seite vom Schaumstoffkörper 13 und auf ihrer von der Durchtrittsöffnung 6 abgelegenen Seite vom Rückenabschnitt 9 begrenzt. Seitlich ist sie im gezeigten Ausführungsbeispiel von den Verbindungsabschnitten 10 begrenzt. Diese seitliche Begrenzung könnte ebenfalls durch den Schaumstoffkörper 13 erfolgen, der hierzu beispielsweise entsprechende in Richtung zum Rückenabschnitt 9 weisende seitliche leistenartige Erhöhungen aufweisen könnte oder von der Hohlkammer 14 aus gesehen konvex gebogen ausgebildet sein könnte. An ihren längsseitigen Enden ist die Hohlkammer 14 von stirnseitigen Wänden 30, 31 begrenzt, die die Hohlkammer 14 an beiden Enden des Bandes 1 verschließen.

Die Wand 11 und der Schaumstoffkörper 13 sind elastisch dehnbar ausgebildet. Die Wand 11 besteht bevorzugterweise aus nicht geschäumtem (vollem) Silikon, ebenso wie der Rückenabschnitt 9 und die gegebenenfalls vorhandenen Verbindungsabschnitte 10.

Die Einrichtung weist weiters ein Anschlussröhrchen 15 auf, dessen innerer Kanal 16 mit der Hohlkammer 14 kommuniziert. Hierzu ist am Band 1 in einem mittleren Abschnitt desselben ein Anschlussstutzen 17 angebracht, der beispielsweise materialeinstückig mit dem Rückenabschnitt 9 ausgebildet sein kann, wobei der Anschlussstutzen 17 über eine Öffnung 18 im Rückenabschnitt 9 mit der Hohlkammer 14 verbunden ist und das Anschlussröhrchen 15 mit einem endseitigen Abschnitt in den Anschlussstutzen 17 ragt und in diesen eingeklebt ist. Das andere Ende des Anschlussröhrchens 15 ist an ein Gefäß 21 angeschlossen, dessen Innenraum durch eine elastisch dehnbare Wand 22 in zwei Kammern 23, 24 unterteilt ist, wobei der Kanal 16 des Anschlussröhrchens 15 mit der Kammer 23 kommuniziert. An das Gefäß 21 ist weiters ein Anschlussröhrchen 25 angeschlossen, dessen innerer Kanal 26 mit der Kammer 24 kommuniziert. Das andere Ende dieses Anschlussröhrchens 25 ist an einen herkömmlichen Injektionsport 19 angeschlossen.

Die Kammer 23 des Gefäßes 21 ist mit Silikongel oder einem anderen körperverträglichen Fluid gefüllt, welches eine höhere Viskosität als Wasser aufweist. Auch die Hohlkammer 14 des Bandes 1 und das Anschlussröhrchen 15 sind mit diesem Füllmedium vorgefüllt. Die Kammer 24 des Gefäßes 21 ist mit einem Druckmedium, beispielsweise sterilem Wasser vorgefüllt.

Nachdem das Band 1 operativ um das entsprechende Körperorgan gelegt worden ist und dem Patienten auch die anderen Teile der Einrichtung implantiert worden sind, wird in den Injektionsport 19 mittels einer Spritze, deren Nadel durch die Haut des Patienten und die Membran 20 des Injektionsportes 19 durchgestochen wurde, mit der gewünschten Menge von sterilem Wasser befüllt, wodurch sich die elastische Wand 22 unter Vergrößerung des Volumens der Kammer 24 und Verringerung des Volumens der Kammer 23 verschiebt. Füllmedium gelangt dadurch in die Hohlkammer 14, wodurch der Schaumstoffkörper 13 und die Wand 11 unter Einengung des Durchlassquerschnittes der Durchtrittsöffnung 6 ausgedehnt werden. Auf diese Weise wird die Durchtrittsöffnung 6 und somit die Größe der Durchlassöffnung des Teils 32 des Gastro-Intestinal-Traktes auf die gewünschte Größe eingestellt.

Stattdessen könnte das Gefäß 21 auch eine Zylinderbohrung aufweisen, in welcher ein die Kammern 23, 24 trennender Kolben angeordnet ist, der eine verschiebbare Wand bildet.

Zur Herstellung des Bandes 1 kann zunächst der durch den Rückenabschnitt 9, die Verbindungsabschnitte 10 und die Wand 11 gebildete umfangsgeschlossene Schlauch hergestellt werden, insbesondere in einem Spritzgussverfahren. Ein Ende dieses Schlauches kann hierbei durch eine stirnseitige Wand 30 geschlossen ausgebildet sein, wobei an dieses Ende auch die entsprechenden Teile der Verschlusseinrichtung 2 bereits angespritzt sind, beispielsweise der Fortsatz 3 mit dem Rastvorsprung 5. In der Folge wird in das offene Ende des Schlauches eine Sprühdüse bis zum geschlossenen Ende des Schlauches eingeführt, deren eine oder mehrere Düsenöffnungen zur Wand 11 gerichtet sind. Unter langsamem Herausziehen der Sprühdüse wird ein Silikonkleber eingesprüht. In der Folge wird der Schaumstoffkörper eingeschoben. Zum Andrücken des Schaumstoffkörpers an die Wand 11 kann ein entsprechender Keil in die Hohlkammer 14 eingeschoben werden. Anschließend oder stattdessen könnte auch eine Flüssigkeit in die Hohlkammer 14 eingefüllt werden. In der Folge wird der Keil aus der Hohlkammer herausgezogen bzw. die Flüssigkeit abgelassen und die Hohlkammer wird am anderen Ende verschlossen, beispielsweise mittels eines Endstückes, welches einen in die Hohlkammer ragenden Pfropf aufweist, der am Schlauch angeklebt wird, wobei am Endstück auch ein Teil der Verschlusseinrichtung 2 angebracht ist.

Eine andere Herstellungsmöglichkeit könnte darin bestehen, den Schaumstoffkörper 13 zunächst in den gespritzten Schlauch einzubringen, ohne vorher Klebstoff einzusprühen. Im Schaumstoffkörper könnten über dessen Länge voneinander beabstandete Durchtrittsöffnungen vorgesehen sein, welche von der Hohlkammer 14 bis zur Wand 11 reichen. In diese Durchtrittsöffnungen könnte nacheinander mittels einer in die Hohlkammer 14 eingeführten und in die Durchtrittsöffnungen eingesteckten Spritzeinrichtung Klebstoff eingedrückt werden, der in den Bereich zwischen dem Schaumstoffring und der Wand 11 jeweils abschnittsweise eindringt und auch die jeweilige Durchtrittsöffnung im Schaumstoffkörper verschließt.

Eine weitere mögliche Ausbildungsform ist in Fig. 6 schematisch dargestellt. Hierbei ist ein separater Rückenabschnitt 9 vorgesehen, von welchem an beiden Seitenrändern sich in Richtung zur Durchtrittsöffnung erstreckende Fortsätze 28 ausgehen. An einen Schaumstoffkörper13 ist eine zumindest dessen zur Durchtrittsöffnung 6 gerichtete Oberfläche überdeckende Haut, vorzugsweise aus Silikon vollflächig angeklebt, wobei sich diese Haut vorzugsweise auch über die gesamte oder zumindest einen Teil der von der Durchtrittsöffnung 6 abgewandten Oberfläche des Schaumstoffkörpers 13 erstreckt. Der an der der Durchtrittsöffnung 6 zugewandten Oberfläche des Schaumstoffkörpers 13 anliegende Abschnitt dieser Haut bildet eine die innere Durchtrittsöffnung 6 begrenzende Wand 11. Der Schaumstoffkörper 13 mit der daran angeklebten Haut wird zwischen den Fortsätzen 28 eingeklebt.

Unterschiedliche Modifikationen der beschriebenen Ausführungsbeispiele der Erfindung sind denkbar und möglich, ohne den Bereich der Erfindung zu verlassen. So könnten beispielsweise das Gefäß 21 auch entfallen und ein Füllmedium, vorzugsweise steriles Wasser, direkt über einen Injektionsport 19 in die Hohlkammer 14 eingebracht werden. Anstelle eines dargestellten Injektionsports könnte auch eine Pumpeinrichtung zur Befüllung der Hohlkammer 14 an das Anschlussröhrchen 15 angeschlossen sein. Eine solche könnte ein Reservoir mit Füllmedium aufweisen, dessen Volumen veränderbar ist. Wenn die Einrichtung als öffen- und schließbarer Verschluss ausgebildet ist, beispielsweise als Analband, so müsste das Reservoir in bekannter Weise zwischen zwei Größen seines Volumens umschaltbar sein, welche dem geschlossenen und dem geöffneten Zustand der Einrichtung entsprechen. Andere Modifikationen könnten beispielsweise die Ausbildung der Wand 11 betreffen, die auch mehrschichtig ausgebildet sein könnte.

## Patentansprüche

1. Einrichtung zur Erzeugung einer künstlichen Verengung im Gastro-Intestinal-Trakt mit einem ringförmig um den jeweiligen Teil des Gastro-Intestinal-Traktes legbaren Band (1) und einer Verschlusseinrichtung (2) zum Verbinden der Endbereiche des ringförmig um den Teil des Gastro-Intestinal-Traktes gelegten Bandes (1), welches im geschlossenen Zustand der Verschlusseinrichtung (2) eine Durchtrittsöffnung (6) umschließt, wobei das Band (1) eine mit einem Füllmedium befüllbare Hohlkammer (14) aufweist, die sich über zumindest einen Großteil der Länge des Bandes (1) erstreckt, und bei einer Befüllung der Hohlkammer (14) eine die Durchtrittsöffnung (6) begrenzende Wand (11) des Bandes (1) sich unter Verkleinerung der Durchtrittsöffnung in Richtung zur Achse (7) der Durchtrittsöffnung ausdehnt, und wobei die Einrichtung weiters einen mit dem Band (1) verbundenen Schaumstoffkörper (13) umfasst, **dadurch gekennzeichnet, dass** der Schaumstoffkörper (13) an die von der Durchtrittsöffnung (6) abgewandte Oberfläche (12) der die Durchtrittsöffnung begrenzenden Wand (11) angrenzt und der Schaumstoffkörper (13) zumindest über einen Großteil seiner an der Wand (11) anliegenden Fläche, vorzugsweise vollflächig, mit der Wand (11) verklebt ist.

2. Einrichtung nach Anspruch 1, **dadurch gekennzeichnet, dass** der Schaumstoffkörper (13) aus Silikonschaum besteht.

3. Einrichtung nach Anspruch 1 oder Anspruch 2, **dadurch gekennzeichnet, dass** die Wand (11) aus Silikon besteht.

4. Einrichtung nach einem der Ansprüche 1 bis 3, **dadurch gekennzeichnet, dass** das Band (1) auf der von der Durchtrittsöffnung (6) abgelegenen Seite einen Rückenabschnitt (9) aufweist, der eine geringere Dehnbarkeit als die Wand (11) aufweist.

5. Einrichtung nach Anspruch 4, **dadurch gekennzeichnet, dass** die Hohlkammer (14) auf ihrer von der Durchtrittsöffnung (6) abgelegenen Seite vom Rückenabschnitt (9) begrenzt ist.

6. Einrichtung nach Anspruch 4 oder Anspruch 5, **dadurch gekennzeichnet, dass** die Wand (11) und der Rückenabschnitt (9) materialeinstückig ausgebildet sind.

7. Einrichtung nach einem der Ansprüche 4 bis 6, **dadurch gekennzeichnet, dass** die Wand (11) und der Rückenabschnitt (9) und gegebenenfalls zwischen dem Rückenabschnitt (9) und der Wand (11) vorhandene Verbindungsabschnitte (10) zusammen einen Schlauch ausbilden.

8. Einrichtung nach einem der Ansprüche 4 bis 7, **dadurch gekennzeichnet, dass** die Dehnbarkeit des Rückenabschnitts (9) geringer als die des Schaumstoffkörpers (13) ist.

9. Einrichtung nach einem der Ansprüche 1 bis 8, **dadurch gekennzeichnet, dass** die von der Durchtrittsöffnung (6) abgewandte Oberfläche (29) des Schaumstoffkörpers (13) die Hohlkammer begrenzt.

10. Einrichtung nach einem der Ansprüche 1 bis 9, **dadurch gekennzeichnet, dass** sich der Schaumstoffkörper (13) über die gesamte Länge der die Durchtrittsöffnung (6) begrenzenden Wand (11) erstreckt und über die gesamte Länge dieser Wand (11) mit ihr verklebt ist.

11. Einrichtung nach einem der Ansprüche 1 bis 10, **dadurch gekennzeichnet, dass** die Einrichtung ein Anschlussröhrchen (15) aufweist, dessen innerer Kanal (16) mit der Hohlkammer (14) kommuniziert.

12. Einrichtung nach einem der Ansprüche 1 bis 11, **dadurch gekennzeichnet, dass** das Füllmedium eine höhere Viskosität als Wasser aufweist, und vorzugsweise Silikongel ist.

13. Einrichtung nach Anspruch 11 oder Anspruch 12, **dadurch gekennzeichnet, dass** das Anschlussröhrchen (15) an einem Gefäß (21) angeschlossen ist, welches eine erste Kammer (23) aufweist, die mit dem Füllmedium gefüllt ist, und eine zweite mit einem Druckmedium gefüllte Kammer (24) aufweist, wobei die beiden Kammern (23, 24) durch eine elastisch dehnbare oder eine verschiebbare Wand (22) voneinander getrennt sind und die mit dem Druckmedium gefüllte Kammer (24) mit einem Injektionsport (19) oder mit einer Pumpeinrichtung verbunden ist.

## Claims

1. A device for the production of an artificial constriction in the gastrointestinal tract, having a band (1) placeable in an annular manner around the specific part of the gastrointestinal tract and a closing device (2) to connect the end regions of the band (1) placed in an annular manner around the part of the gastrointestinal tract, which band (1) in the closed state of the closing device (2) surrounds an opening (6), wherein the band (1) has a hollow chamber (14), fillable with a filling medium, which extends over at least a major part of the length of the band (1), and upon filling of the hollow chamber (14), a wall (11), of the band (1), bounding the opening (6) stretches in the direction of the axis (7) of the opening accompanied by a reduction in size of the opening, and wherein the device furthermore comprises a body (13) of cellular material connected to the band (1), **characterised in that** the cellular material body (13) adjoins the surface (12), remote from the opening (6), of the wall (11) bounding the opening and the cellular material body (13) is stuck, preferably over its entire surface, to the wall (11) over at least a major part of its face contacting the wall (11).

2. A device according to claim 1, **characterised in that** the cellular material body (13) is composed of silicone foam.

3. A device according to claim 1 or claim 2, **characterised in that** the wall (11) is composed of silicone.

4. A device according to any one of claims 1 to 3, **characterised in that** on its side distant from the opening (6), the band (1) has a back portion (9) of less extensibility than the wall (11).

5. A device according to claim 4, **characterised in that** on its side distant from the opening (6), the hollow chamber (14) is bounded by the back portion (9).

6. A device according to claim 4 or claim 5, **characterised in that** the wall (11) and the back portion (9) are in one piece of material.

7. A device according to any one of claims 4 to 6, **characterised in that** the wall (11) and the back portion (9) and connecting portions (10) optionally present between the back portion (9) and the wall (11) together form a hose.

8. A device according to any one of claims 4 to 7, **characterised in that** the extensibility of the back portion (9) is less than that of the cellular material body (13).

9. A device according to any one of claims 1 to 8, **characterised in that** the surface (29), remote from the opening (6), of the cellular material body (13) bounds the hollow chamber.

10. A device according to any one of claims 1 to 9, **characterised in that** the cellular material body (13) extends over the entire length of the wall (11) bounding the opening (6) and is stuck to this wall (11) over the entire length of the latter.

11. A device according to any one of claims 1 to 10, **characterised in that** the device has a connection tube (15) whose inner channel (16) communicates with the hollow chamber (14).

12. A device according to any one of claims 1 to 11, **characterised in that** the filling medium has a higher viscosity than water and is preferably silicone gel.

13. A device according to claim 11 or claim 12, **characterised in that** the connection tube (15) is attached to a container (21) which has a first chamber (23) filled with the filling medium and a second chamber (24) filled with a pressure medium, the two chambers (23, 24) being separated from one another by a resiliently extensible or a displaceable wall (22) and the chamber (24) filled with the pressure medium being connected to an injection port (19) or to a pumping device.

## Revendications

1. Dispositif pour générer une constriction artificielle de l'appareil gastro-intestinal avec une bande (1) à poser en forme d'anneau autour de la partie respective de l'appareil gastro-intestinal et un dispositif de fermeture (2) pour assembler les zones d'extrémité de la bande (1), posée en forme d'anneau autour de la partie de l'appareil gastro-intestinal, qui entoure un orifice de passage (6) lorsque le dispositif de fermeture (2) se trouve à l'état fermé,
la bande (1) présente sur la majeure partie de sa longueur une chambre creuse (14) pouvant être remplie d'un fluide, et lors d'un remplissage de la chambre creuse (14) une paroi (11) de la bande (1) limitant l'orifice de passage (6) se dilate en direction de l'axe (7) de l'orifice de passage en le rétrécissant, et
le dispositif comprend en outre un corps en mousse (13) assemblé à la bande (1),
**caractérisé en ce que**
le corps de mousse (13) est adjacent à la surface (12) de la paroi (11) limitant l'orifice de passage (6) située à l'opposé de celui-ci et il est collé à la paroi (11) au moins sur une grande partie de sa surface, de préférence sur toute sa surface.

2. Dispositif selon la revendication 1,
**caractérisé par**
le corps de mousse (13) en mousse de silicone.

3. Dispositif selon la revendication 1 ou 2,
**caractérisé par**
la paroi (11) en silicone.

4. Dispositif selon l'une quelconque des revendications 1 à 3,
**caractérisé en ce que**
la bande (1) comporte, sur son côté éloigné de l'orifice de passage (6), une partie dorsale (9) présentant une dilatabilité inférieure à celle de la paroi (11).

5. Dispositif selon la revendication 4,
**caractérisé en ce que**
la chambre creuse (14) est limitée par la partie dorsale (9) sur son côté éloigné de l'orifice de passage (6).

6. Dispositif selon la revendication 4 ou 5,
**caractérisé en ce que**
la paroi (11) et la partie dorsale (9) sont réalisées en continuité de matière.

7. Dispositif selon l'une des revendications 4 à 6,
**caractérisé en ce que**
la paroi (11), la partie dorsale (9) et éventuellement des parties d'assemblage présentes entre elles forment ensemble un tuyau souple.

8. Dispositif selon l'une des revendications 4 à 7,
**caractérisé en ce que**
la dilatabilité de la partie dorsale (9) est inférieure à celle du corps de mousse (13).

9. Dispositif selon l'une des revendications 1 à 8,
**caractérisé en ce que**
la surface (29) du corps de mousse (13) située à l'opposé de l'orifice de passage (6) délimite la chambre creuse.

10. Dispositif selon l'une des revendications 1 à 9,
**caractérisé en ce que**
le corps de mousse (13) s'étend sur toute la longueur de la paroi (11) délimitant l'orifice de passage (6) et est collé à cette paroi (11) sur toute sa longueur.

11. Dispositif selon l'une des revendications 1 à 10,
**caractérisé en ce que**
le dispositif comprend un petit tube de raccordement (15), dont le canal intérieur (16) communique avec la chambre creuse (14).

12. Dispositif selon l'une des revendications 1 à 11,
**caractérisé en ce que**
le fluide de remplissage présente une viscosité supérieure à celle de l'eau, et est de préférence un gel de silicone.

13. Dispositif selon la revendication 11 ou 12,
**caractérisé en ce que**
le petit tube de raccordement (15) est raccordé à un réservoir (21), qui présente une première chambre (23), remplie du fluide de remplissage, et une deuxième chambre (24) remplie d'un fluide sous pression, les deux chambres (23, 24) étant séparées l'une de l'autre par une paroi élastiquement déformable ou déplaçable (22) et la chambre (24) remplie du fluide sous pression est raccordée à un port d'injection (19) ou à un dispositif de pompe.
